# EUROPÄISCHE PATENTSCHRIFT

(11) **EP 1 338 593 B1**
(45) Veröffentlichungstag und Bekanntmachung des Hinweises auf die Patenterteilung: **01.12.2004**
(21) Anmeldenummer: 03002606.6
(22) Anmeldetag: 10.02.2003
(51) Int. Cl.: C07D 309/30

(54) **Verfahren zur Herstellung von 3,6-Dihydro-2H-pyran-2-carbonsäureestern**
Process for the preparation of 3,6-dihydro-2H-pyran-2-carbonic acid esters
Procédé pour la préparation des esters d'acide 3,6-dihydro-2H-pyran-2-carbonique acide

(30) Priorität: 21.02.2002 DE 10207410
(43) Veröffentlichungstag der Anmeldung: 27.08.2003
(73) Patentinhaber: Bayer Chemicals AG, 51368 Leverkusen (DE)
(72) Erfinder: Lögers, M., Dr., 42327 Wuppertal (DE)

(56) Entgegenhaltungen:
- JOHANNSEN M ET AL: "ASYMMETRIC HETERO DIELS-ALDER REACTIONS AND ENE REACTIONS CATALYZED BY CHIRAL COPPER(II) COMPLEXES" JOURNAL OF ORGANIC CHEMISTRY, AMERICAN CHEMICAL SOCIETY. EASTON, US, Bd. 18, Nr. 60, September 1995 (1995-09), Seiten 5757-5762, XP001083632 ISSN: 0022-3263
- ROBERT H ET AL: "The Carbonyl-Diels-Alder Reaction Catalyzed by Bismuth (III) Chloride" TETRAHEDRON LETTERS, ELSEVIER SCIENCE PUBLISHERS, AMSTERDAM, NL, Bd. 39, Nr. 10, 5. März 1998 (1998-03-05), Seiten 1161-1164, XP004109144 ISSN: 0040-4039

## Beschreibung

Die Anmeldung betrifft ein Verfahren zur Herstellung von 3,6-Dihydro-2H-pyran-2-carbonsäureestem über eine thermische Hetero Diels-Alder-Reaktion aus 1,3-Dienen und Estern der Glyoxylsäure.

Die Hetero Diels-Alder-Reaktion von konjugierten Dienen mit Carbonylverbindungen ist eine der fundamentalen Reaktionen in der organischen Chemie.

Konjugierte Diene lassen sich prinzipiell mit Glyoxylsäureestern thermisch in einer Hetero Diels-Alder-Reaktion zu 3,6-Dihydro-2H-pyran-2-carbonsäureestem umsetzen.

Aus J. Org. Chem. USSR, 1970, 6, 411 ist es bekannt, 2,3-Dimethyl-1,3-butadien oder Isopren mit Ethylglyoxylat in einer thermischen Hetero Diels-Alder-Reaktion zu den entsprechenden 3,6-Dihydro-2H-pyran-2-carbonsäureestem umzusetzen. Hierzu werden im 500 mmol-Maßstab Dien, Ethylglyoxylat und Hydrochinon gemischt und anschließend auf 130°C erwärmt. Die 3,6-Dihydro-2H-pyran-2-carbonsäureester werden in diesem Labormaßstab in Ausbeuten von 40 bis 74 % erhalten.

Gemäß Tetrahedron 1993, 49, 4639-4650 werden 10 mmol ({[(3E)-2-(benzyloxy)-3,5-hexadienyl]oxy}methyl)benzol sowie andere Diene mit Butylglyoxylat und Hydrochinon gemischt und über 18 Stunden auf 130°C erwärmt. Nach Aufarbeitung werden Cycloaddukte in 60 %iger Ausbeute erhalten.

Auch aus Org. Magn. Res. 1983, 21, 94-107 ist die Herstellung von 3,6-Dihydro-2Hpyran-2-carbonsäure-butylestern über eine thermische Diels-Alder-Reaktion aus 1,3-Butadien und Butylglyoxylat in kleinem Maßstab bekannt.

Höher substituierte, konjugierte Diene reagieren im Allgemeinen bei niedrigeren Temperaturen und in besseren Ausbeuten. So nehmen beispielsweise Reaktivität und Ausbeute in der Reihenfolge 1,3-Butadien < 2-Methyl-1,3-butadien < 2,3-Dimethyl-1,3-butadien zu.

In jüngerer Zeit wurden Fortschritte auf dem Weg der katalysierten Hetero-Diels-Alder-Reaktionen mit Glyoxylsäureestem gemacht. Vorteil dieser, beispielsweise in Tetrahedron Lett. 1998, 39, 1161-1-164, J. Chem. Soc. Chem. Commun. 1996, 2373-2374, J. Chem. Soc. Perkin Trans 1, 1997, 2345-2349 und J. Org. Chem. 1995, 60, 5757-5762 beschriebenen Methoden ist häufig eine niedrigere Reaktionstemperatur. Nachteil der katalysierten Synthesen ist, dass teuere (BiCl₃, Me-Al[(S)-BINOL], Zn(OTf)₂), toxische (SnCl₂, Cu-Verbindungen) und oft schwer herzustellende Katalysatoren verwendet werden, die das Verfahren unwirtschaftlich machen. Zusätzlich ist die Abtrennung des Produktes deutlich aufwendiger. Für die meisten dieser Beispiele ist eine großtechnische Umsetzung auch aus Umweltschutzgründen nicht möglich, da Schwermetallabfälle (Bi, Sn, Cu) anfallen.

In J. Org. Chem. 1995, 60, 5757-5762 wird die durch einen Kupfer(II)-bisoxazolin-Komplex katalysierte Reaktion von Glyoxylsäureestern mit konjugierten Dienen beschrieben. Hierbei wird Isopropylglyoxylat beispielsweise im 10 mmol-Maßstab mit 5 bis 10 Äquivalenten 1,3-Butadien in Dichlormethan in Gegenwart eines Katalysators, der in situ aus Cu(OTf)₂ und einem chiralen Oxazolidinon gebildet wird, innerhalb von 5 Tagen zu dem entsprechenden 3,6-Dihydro-2H-pyran-2-carbonsäureester umgesetzt. Die erzielte Ausbeute liegt bei 55%.

Allen zuvor genannten Veröffentlichungen zur thermisch katalysierten Umsetzung konjugierter Diene mit Glyoxylsäureestem zu 3,6-Dihydro-2H-pyran-2-carbonsäureestern ist der Nachteil gemeinsam, dass die Umsetzung eine Vorgehensweise umfasst, die im Rahmen der vorliegenden Anmeldung als "Batch-Verfahren" bezeichnet wird. Bei diesem "Batch-Verfahren" werden alle Reaktionskomponenten, d.h. das Dien und das Glyoxylat (sowie der üblicherweise zugesetzte Stabilisator) zunächst bei niedriger Temperatur unmittelbar zusammengegeben und miteinander vermischt und anschließend durch Erhitzen auf die benötigte Reaktionstemperatur von in der Regel mehr als 100°C gebracht. Diese "Batch-Verfahren" sind nur für eine Durchführung im Labormaßstab beschrieben.

Diese Vorgehensweise ist jedoch insbesondere bei einer Umsetzung im größeren Maßstab sicherheitstechnisch ausgesprochen bedenklich, da bekannt ist, dass konjugierte Diene, speziell 1,3-Butadien, bei erhöhter Temperatur zur thermischen Polymerisation neigen und die damit verbundene adiabate Temperaturerhöhung zur thermischen Zersetzung bis hin zur Explosion der Reaktionsgemische führen kann.

Die stark exotherme Polymerisation von 1,3-Butadien setzt beispielsweise bei Temperaturen oberhalb 100°C mit einer Wärmetönung von etwa 1350 kJ/kg ein. Unmittelbar im Anschluss an die Polymerisation setzt ab etwa 295°C eine stark exotherme Zersetzung des Polymerisates ein. Die Wärmefreisetzung der Zersetzung beträgt ca. 1500 kJ/kg (Odian "Principles of Polymerization" S. 264- Table 3-14.)

Diese Faktoren führen zu einem Anstieg der Reaktionsgeschwindigkeit und einem exponentiellen Anstieg der Wärmeleistung. Die Folge ist eine nicht mehr zu kontrollierende, exotherme Reaktion (sogenannte "Runaway"-Reaktion). Dies steht insbesondere einer Übertragung dieser Reaktionen in den großtechnischen Maßstab und damit einer wirtschaftlichen Nutzung dieses prinzipiellen Syntheseweges entgegen.

Die Aufgabe der vorliegenden Erfindung bestand somit darin, ein Verfahren zur Herstellung von 3,6-Dihydro-2H-pyran-2-carbonsäureestem über eine thermische Hetero Diels-Alder-Reaktion aus 1,3-Dienen bereitzustellen, welches kein inhärentes Sicherheitsproblem wie die bekannten "Batch-Verfahren" aufweist und damit eine Synthese in größeren Maßstäben bei gleichzeitig guten Ausbeuten des gewünschten Produktes ermöglicht.

Gegenstand der vorliegenden Anmeldung ist ein Verfahren zur Herstellung von 3,6-Dihydro-2H-pyran-2-carbonsäureestem der allgemeinen Formel (I), 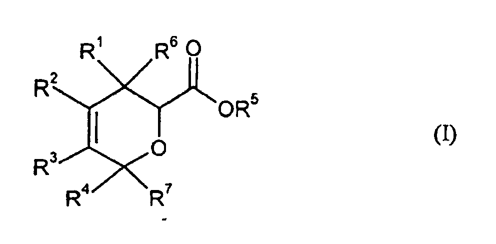 worin
- R¹, R², R³ und R⁴: gleich oder verschieden sind und H oder einen geradkettigen oder verzweigten C₁-C₇ -Alkylrest bedeuten,
- R⁵: einen geradkettigen oder verzweigten C₁-C₇-Alkylrest, einen Phenylrest oder einen Benzylrest bedeutet,
- R⁶ und R⁷: gleich oder verschieden sind und H oder geradkettiges oder verzweigtes C₁-C₃-Alkyl bedeuten oder gemeinsam einen - (CH₂)ₙ- Alkylenrest bilden, bei dem n= 1 oder 2 ist,
über eine thermische Hetero Diels-Alder-Reaktion von 1,3-Dienen der allgemeinen Formel (II), 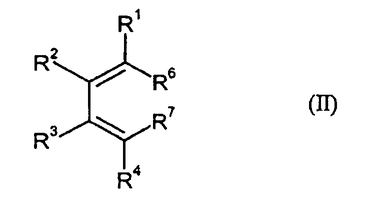 worin
- R¹, R², R³, R⁴, R⁶ und R⁷: die für die allgemeine Formel (I) genannten Bedeutungen haben, mit Verbindungen der allgemeinen Formel (III) 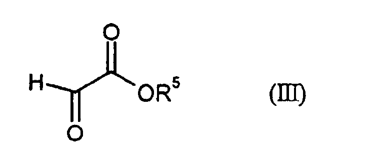
worin
- R⁵: die für die allgemeine Formel (I) genannten Bedeutungen hat, dadurch gekennzeichnet, dass das Verfahren in Abwesenheit von Katalysatoren und Stabilisatoren durchgeführt wird und zunächst die Verbindung der allgemeinen Formel (III) vorgelegt und auf eine Temperatur von 90-230°C gebracht wird und anschließend das 1,3-Dien der allgemeinen Formel (II) zugegeben wird.

Bei den im erfindungsgemäßen Verfahren eingesetzten Verbindungen der allgemeinen Formel (II) handelt es sich bevorzugt um solche, bei denen die Reste R¹, R² , R³ und R⁴ gleich oder verschieden sind und für Wasserstoff, Methyl, Ethyl, n-Propyl, iso-Propyl, n-Butyl, iso-Butyl, tert.-Butyl oder n-Pentyl stehen. R⁶ und R⁷ stehen bevorzugt, gleich oder verschieden, für Wasserstoff oder Methyl.

Besonders bevorzugt stehen die Reste R¹, R², R³ und R⁴ für Wasserstoff und auch R⁶ und R⁷ für Wasserstoff. Alternativ bevorzugt steht mindestens einer der Reste R¹, R², R³ oder R⁴, besonders bevorzugt einer oder zwei der Reste R¹, R², R³ oder R⁴ für Methyl und R⁶ und R⁷ gleichzeitig , gleich oder verschieden, für Wasserstoff oder Methyl.

Bei den im erfindungsgemäßen Verfahren eingesetzten Verbindungen der allgemeinen Formel (III) handelt es sich bevorzugt um solche, bei denen der Rest R⁵ Methyl, Ethyl, n-Propyl, iso-Propyl, n-Butyl, iso-Butyl, tert.-Butyl, n-Pentyl, Phenyl oder Benzyl bedeutet. Besonders bevorzugt steht R⁵ für Methyl, Ethyl, n-Propyl, iso-Propyl, n-Butyl, iso-Butyl oder tert.Butyl.

Steht R⁵ für einen Phenyl- oder Benzylrest, so kann dieser ein-, zwei-, drei-, vieroder fünffach substituiert sein. Als Substituenten des Phenyl- oder Benzylrestes sind beispielsweise C₁-C₄-Alkyl, bevorzugt CH₃ oder C₂H₅, Halogen, bevorzugt Fluor, Chlor oder Brom, Nitro, -OC₁-C₄-Alkyl, bevorzugt -OCH₃ oder -OC₂H₅, und -COOC₁-C₄-Alkyl Reste, bevorzugt -COOCH₃ oder -COOC₂H₅ geeignet.

Verglichen mit dem Stand der Technik zeichnet sich das erfindungsgemäße Verfahren durch eine spezielle Dosiertechnik aus. Die Verbindung der allgemeinen Formel (III) wird vorgelegt und auf die Reaktionstemperatur von 90 bis 230°C gebracht. Erst danach erfolgt die Zugabe des 1,3-Diens der allgemeinen Formel (II). Üblicherweise erfolgt die Zugabe des 1,3-Diens so, dass im Reaktionsgemisch jeweils nur ein geringer Überschuss an 1,3-Dien vorliegt.

Überraschenderweise lässt sich bei dieser Art der Durchführung des erfindungsgemäßen Verfahrens völlig auf die Anwesenheit von teuren, toxischen und umweltbelastenden Schwermetallkatalysatoren verzichten. Auch der Einsatz von Stabilisatoren, wie Hydrochinon, Chinon, Phenothiazin, Pikrinsäure, TBC (p-Tert-butylpyrocatechol), TBK (2,6-Di-tert-butyl-p-cresol) oder 2,6-Di-tert.-butyl-4-methylphenol ist nicht mehr erforderlich. Gemäß dem bekannten Stand der Technik werden diese Stabilisatoren immer verwendet, wobei Mengen von 0,1 bis 5 Gew. %, bezogen auf das 1,3-Dien, eingesetzt werden.

Es hat sich bewährt, das erfindungsgemäße Verfahren so durchzuführen, dass die Verbindung der allgemeinen Formel (III), d.h. der Glyoxylsäureester, als solcher oder gelöst in einem inerten Lösungsmittel im Reaktionsbehältnis vorgelegt wird. Als inerte Lösungsmittel können beispielsweise Pentan, Cyclohexan, Hexan, Benzol, Toluol, Xylole, Petrolether, Chlorbenzol oder Dichlorbenzole eingesetzt werden.

Anschließend wird auf die Reaktionstemperatur im Bereich von 90 bis 230°C, bevorzugt im Bereich von 100 bis 180°C erwärmt. Je nach eingesetztem 1,3-Dien ist auch der Bereich von 120 bis 160°C besonders bevorzugt. Bei Einsatz von 1,3-Butadien haben sich Reaktionstemperaturen bis maximal 180°C bewährt.

Wenn niedrigsiedende Verbindungen als Edukte des erfindungsgemäßen Verfahrens eingesetzt werden, d.h. Edukte mit einem Siedepunkt von bis zu 100°C, insbesondere 1,3-Butadien, Isopren und 2,3-Dimethyl-1,3-butadien, so wird als Reaktionsbehältnis üblicherweise ein Autoklav verwendet, der mindestens bis 25 bar druckfest ist. Nach Vorlage der Verbindung der allgemeinen Formel (III) wird dieser Autoklav druckdicht verschlossen. Durch die Erhöhung der Temperatur auf die Reaktionstemperatur steigt der Innendruck des geschlossenen Autoklaven je nach Befüllungsgrad auf 2 bis 10 bar an. Aus sicherheitstechnischen Gründen werden üblicherweise maximal 80 % des Nennreaktorvolumens befüllt. Bei Einsatz von ausschließlich höhersiedenden Edukten im erfindungsgemäßen Verfahren können dagegen auch Rührwerksbehälter zur Anwendung gelangen: Liegen die Siedepunkte der Edukte oberhalb von 100°C, so wird dieser Rührwerksbehälter in druckdicht verschlossener Form verwendet. Bei Edukten mit Siedepunkten oberhalb von 200°C bzw. oberhalb der Reaktionstemperatur kann auch mit einem offenen Rührwerksbehälter gearbeitet werden.

Nach Erreichen der Reaktionstemperatur wird anschließend das 1,3-Dien der allgemeinen Formel (II) in einer Menge von 1.0 bis 3.0 Äquivalenten, bevorzugt 1.3 bis 1.5 Äquivalenten, bezogen auf die Verbindung der allgemeinen Formel (III), in das Reaktionsbehältnis zugegeben. Im Fall der Verwendung gasförmiger 1,3-Diene wie 1,3-Butadien erfolgt diese Zugabe üblicherweise aus einem Drucktank gegen den im Reaktionsbehältnis entstandenen Innendruck durch Hineindrücken entweder mittels Stickstoff oder mittels einer Dosierpumpe.

Die Dosierzeit für die Zugabe des 1,3-Diens der allgemeinen Formel (II) beträgt 3 bis 36 Stunden. Die jeweils verwendete Dosierzeit hängt von dem gewählten 1,3-Dien sowie der Reaktionstemperatur ab. Bei einer Reaktionstemperatur im Bereich von 150-180°C hat sich eine Reaktionszeit von 16 bis 6 Stunden bewährt. Daran schließt sich geeigneterweise eine Nachreaktion von 3 bis 24 Stunden bei der zuvor genannten Reaktionstemperatur an. Üblicherweise erfolgt die Nachreaktion unter Rühren.

Anschließend wird das Reaktionsgemisch auf 0 bis 70°C, bevorzugt auf 20 bis 40°C, abgekühlt und aus dem Reaktionsbehältnis entnommen. Üblicherweise erfolgt eine Überführung des Inhaltes des Reaktionsbehältnisses in eine Destillationsanlage. Bei der Destillation, die entweder unter Normaldruck oder aber auch unter vermindertem Druck durchgeführt wird, werden Lösungsmittel, nicht-umgesetzte Edukte und Nebenkomponenten von dem gewünschten 3,6-Dihydro-2H-pyran-2-carbonsäureester destillativ abgetrennt. Sofern gewünscht, kann die Destillation unter Einsatz von Kolonnen durchgeführt werden. Möglich ist aber auch die Durchführung in Form einer Dünnschichtdestillation oder über einen Kurzwegverdampfer.

Das erfindungsgemäße Verfahren erlaubt aufgrund seiner speziellen Dosiertechnik nicht nur das Weglassen der Katalysatoren sowie Stabilisatoren, sondern ermöglicht eine sicherheitstechnisch ungefährliche Durchführung der Reaktion selbst im großtechnischen Tonnen-Maßstab.

Gleichzeitig erhält man bei Wahl der gleichen Edukte eine gegenüber den bekannten Verfahren des Standes der Technik höhere Ausbeute an den gewünschten 3,6-Dihydro-2H-pyran-2-carbonsäureestem von 30 bis 80 %. Durch die besondere Dosiertechnik ist jeweils nur ein geringer Überschuss an 1,3-Dien im System vorhanden, wodurch auch die allmähliche Polymerisation des 1,3-Diens zu unerwünschten "Popcom"-Polymeren vermindert wird.

## Patentansprüche

1. Verfahren zur Herstellung von 3,6-Dihydro-2H-pyran-2-carbonsäureestem der allgemeinen Formel (I), 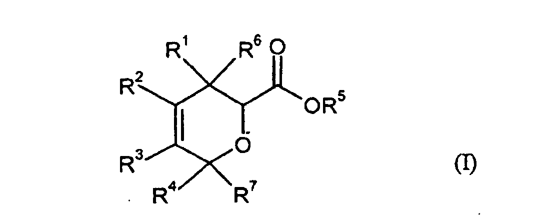 worin
R¹, R², R³ und R⁴ gleich oder verschieden sind und H oder einen geradkettigen oder verzweigten C₁-C₇-Alkylrest bedeuten,
R⁵ einen geradkettigen oder verzweigten C₁-C₇-Alkylrest, einen Phenylrest oder einen Benzylrest bedeutet,
R⁶ und R⁷ gleich oder verschieden sind und H oder geradkettiges oder verzweigtes C₁-C₃-Alkyl bedeuten oder gemeinsam einen -(CH₂)ₙ- Alkylenrest bilden, bei dem n= 1 oder 2 ist,
über eine thermische Hetero Diels-Alder-Reaktion von 1,3-Dienen der allgemeinen Formel (II), 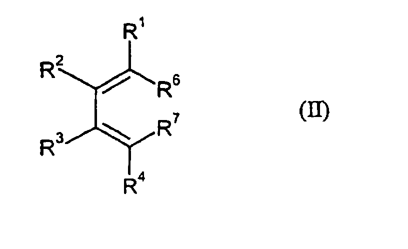 worin
R¹, R², R³, R⁴, R⁶ und R⁷ die für die allgemeine Formel (I) genannten Bedeutungen haben, mit Verbindungen der allgemeinen Formel (III), 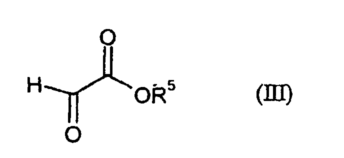
worin
R⁵ die für die allgemeine Formel (I) genannten Bedeutungen hat, **dadurch gekennzeichnet, dass** das Verfahren in Abwesenheit von Katalysatoren und Stabilisatoren durchgeführt wird und zunächst die Verbindung der allgemeinen Formel (III) vorgelegt und auf eine Temperatur von 90-230°C gebracht wird und anschließend das 1,3-Dien der allgemeinen Formel (II) zugegeben wird.

2. Verfahren gemäß Anspruch 1, **dadurch gekennzeichnet, dass** als Verbindungen der allgemeinen Formel (II) solche eingesetzt werden, bei denen die Reste R¹, R², R³ und R⁴ gleich oder verschieden sind und für Wasserstoff, Methyl, Ethyl, n-Propyl, iso-Propyl, n-Butyl, iso-Butyl, tert.-Butyl oder n-Pentyl stehen.

3. Verfahren gemäß Anspruch 1, **dadurch gekennzeichnet, dass** die Reste R¹, R², R³ R⁴, R⁶ und R⁷ für Wasserstoff stehen.

4. Verfahren gemäß einem oder mehreren der Ansprüche 1 bis 3, **dadurch gekennzeichnet, dass** als Verbindungen der allgemeinen Formel (III) solche eingesetzt werden, bei denen der Rest R⁵ Methyl, Ethyl, n-Propyl, iso-Propyl, n-Butyl, iso-Butyl, tert.-Butyl, n-Pentyl, Phenyl oder Benzyl bedeutet.

5. Verfahren gemäß einem oder mehreren der Ansprüche 1 bis 4, **dadurch gekennzeichnet, dass** die Verbindung der allgemeinen Formel (III) als solche oder gelöst in einem inerten Lösungsmittel vorgelegt wird.

6. Verfahren gemäß einem oder mehreren der Ansprüche 1 bis 5, **dadurch gekennzeichnet, dass** auf eine Temperatur im Bereich von 100 bis 180 °C erwärmt wird.

7. Verfahren gemäß einem oder mehreren der Ansprüche 1 bis 6, **dadurch gekennzeichnet, dass** das 1,3-Dien der allgemeinen Formel (II) in einer Menge von 1.0 bis 3.0 Äquivalenten, bevorzugt 1.3 bis 1.5 Äquivalenten, bezogen auf die Verbindung der allgemeinen Formel (III), zugegeben wird.

8. Verfahren gemäß einem oder mehreren der Ansprüche 1 bis 7, **dadurch gekennzeichnet, dass** die Zugabe des 1,3-Diens der allgemeinen Formel (II) so erfolgt, dass im Reaktionsgemisch jeweils nur ein geringer Überschuss an 1,3-Dien vorliegt.

9. Verfahren gemäß einem oder mehreren der Ansprüche 1 bis 8, **dadurch gekennzeichnet, dass** die Dosierzeit für die Zugabe des 1,3-Diens der allgemeinen Formel (II) 3 bis 36 Stunden beträgt.

## Claims

1. Process for preparing 3,6-dihydro-2H-pyran-2-carboxylic esters of the general formula (I) 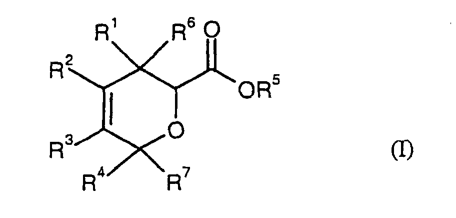 where
R¹, R² , R³ and R⁴ are the same or different and are each H or a straight-chain or branched C₁-C₇-alkyl radical,
R⁵ is a straight-chain or branched C₁-C₇-alkyl radical, a phenyl radical or a benzyl radical,
R⁶ and R⁷, are the same or different and are each H or straight-chain or branched C₁-C₃-alkyl or together form a - (CH₂)ₙ- alkylene radical where n = 1 or 2,
via a thermal hetero Diets-Alder reaction of 1,3-dienes of the general formula (II) 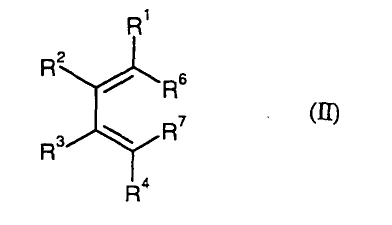 where
R¹, R², R³, R⁴, R⁶ and R⁷ are each as defined for the general formula (I) with compounds of the general formula (III) 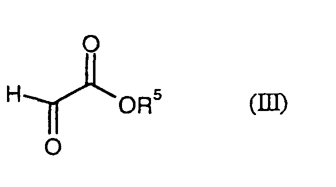
where
R⁵ is as defined for the general formula (I), **characterized in that** the process is carried out in the absence of catalysts and stabilizers and the compound of the general formula (III) is initially charged and brought to a temperature of 90-230°C and the 1,3-diene of the general formula (II) is then added.

2. Process according to Claim 1, **characterized in that** the compounds of the general formula (II) used are those in which the R¹, R² , R³ and R⁴ radicals are the same or different and are each hydrogen, methyl, ethyl, n-propyl, isopropyl, n-butyl, isobutyl, tert-butyl or n-pentyl.

3. Process according to Claim 1, **characterized in that** the R¹, R², R³ R⁴, R⁶ and R⁷ radicals are each hydrogen.

4. Process according to one or more of Claims 1 to 3, **characterized in that** the compounds of the general formula (III) used are those in which the R⁵ radical is methyl, ethyl, n-propyl, isopropyl, n-butyl, isobutyl, tert-butyl, n-pentyl, phenyl or benzyl.

5. Process according to one or more of Claims 1 to 4, **characterized in that** the compound of the general formula (III) is used as such or initially charged dissolved in an inert solvent.

6. Process according to one or more of Claims 1 to 5, **characterized in that** heating is effected to a temperature in the range from 100 to 180°C.

7. Process according to one or more of Claims 1 to 6, **characterized in that** the 1,3-diene of the general formula (II) is used in an amount of 1.0 to 3.0 equivalents, preferably 1.3 to 1.5 equivalents, based on the compound of the general formula (III).

8. Process according to one or more of Claims 1 to 7, **characterized in that** the 1,3-diene of the general formula (II) is added in such a manner that there is in each case only a small excess of 1,3-diene in the reaction mixture.

9. Process according to one or more of Claims 1 to 8, **characterized in that** the metering time for the addition of the 1,3-diene of the general formula (II) is 3 to 36 hours.

## Revendications

1. Procédé de préparation d'esters d'acide 3,6-dihydro-2H-pyran-2-carbonique de formule générale (I), 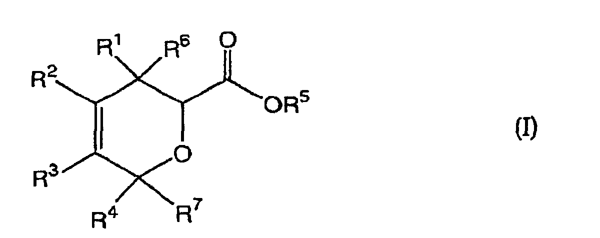 dans laquelle
R¹, R², R³ et R⁴ sont identiques ou différents et signifient H ou un radical alkyle en C₁-C₇ linéaire ou ramifié,
R⁵ signifie un radical alkyle en C₁-C₇ linéaire ou ramifié, un radical phényle ou un radical benzyle,
R⁶ et R⁷ sont identiques ou différents et signifient H ou un radical alkyle en C₁-C₃ linéaire ou ramifié ou ensemble forment un radical alkylène -(CH₂)ₙ-, dans lequel n = 1 ou 2,
via une réaction de Diels-Alder hétérogène thermique des 1,3-diènes de formule générale (II), 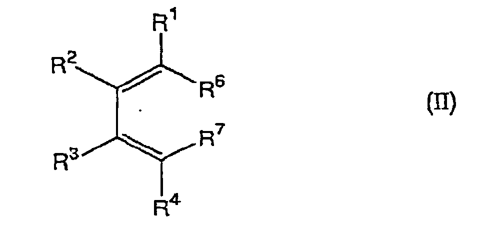 dans laquelle
R¹, R², R³, R⁴, R⁶ et R⁷ ont les significations données pour la formule générale (I), avec des composés de formule générale (III), 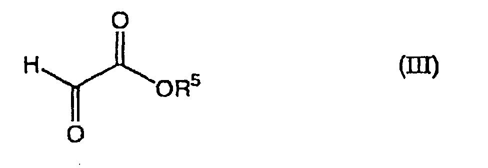 dans laquelle
R⁵ a les significations données pour la formule générale (I), **caractérisé en ce qu'**on effectue le procédé en absence de catalyseurs et de stabilisants et qu'on introduit d'abord le composé de formule générale (III) et qu'on le porte à une température de 90 - 230 °C et ensuite qu'on ajoute le 1,3-diène de formule générale (II).

2. Procédé selon la revendication 1, **caractérisé en ce qu'**on utilise comme composés de formule générale (II) ceux pour lesquels les radicaux R¹, R², R³ et R⁴ sont identiques ou différents et sont un hydrogène, un radical méthyle, éthyle, n-propyle, iso-propyle, n-butyle, iso-butyle, tert.-butyle ou n-pentyle.

3. Procédé selon la revendication 1, **caractérisé en ce que** les radicaux R¹, R², R³, R⁴ R⁶ et R⁷ sont l'hydrogène.

4. Procédé selon une ou plusieurs des revendications 1 à 3, **caractérisé en ce qu'**on utilise comme composés de formule générale (III) ceux dans lesquels le radical R⁵ signifie un radical méthyle, éthyle, n-propyle, iso-propyle, n-butyle, iso-butyle, tert.-butyle, n-pentyle, phényle ou benzyle.

5. Procédé selon une ou plusieurs des revendications 1 à 4, **caractérisé en ce qu'**on utilise tel quel le composé de formule générale (III) ou dissous dans un solvant inerte.

6. Procédé selon une ou plusieurs des revendications 1 à 5, **caractérisé en ce qu'**on chauffe à une température dans la plage de 100 à 180 °C.

7. Procédé selon une ou plusieurs des revendications 1 à 6, **caractérisé en ce qu'**on ajoute le 1,3-diène de formule générale (II) en une quantité de 1,0 à 3,0 équivalents, de préférence 1,3 à 1,5 équivalents, rapporté au composé de formule générale (III).

8. Procédé selon une ou plusieurs des revendications 1 à 7, **caractérisé en ce qu'**on effectue l'addition du 1,3-diène de formule générale (II) de manière que dans le mélange réactionnel se trouve chaque fois seulement un faible excès de 1,3-diène.

9. Procédé selon une ou plusieurs des revendications 1 à 8, **caractérisé en ce que** la durée de dosage pour l'addition du 1,3-diène de formule générale (II) est de 3 à 36 heures.
